# EUROPEAN PATENT APPLICATION

(11) **EP 2 371 289 A1**
(43) Date of publication of application: **05.10.2011**
(21) Application number: 09826206.6
(22) Date of filing: 18.02.2009
(51) Int. Cl.: A61B 6/14

(54) **FILM HOLDER WITH ANGLE INDICATOR FOR INTRAORAL RADIOGRAPHY AND FILM SUPPORTING PART THEREOF**

(30) Priority: 17.11.2008 KR 20080114181
(71) Applicant: Choi, Young Jin, Gyeonggi-do 410-380 (KR)
(72) Inventor: Choi, Young Jin, Gyeonggi-do 410-380 (KR)
(74) Representative: Rupprecht, Kay
(86) International application number: PCT/KR2009/000766
(87) International publication number: WO 2010/055973

(57) **Abstract**

The film holder located at a lingual side of the teeth inside mouth to support a film for periapical radiography to obtain the radiographic image of the teeth and the teeth supporting structures comprises a bite portion temporarily fixed to the teeth; a film support portion rotatably mounted with respect to the bite portion and located at the lingual side of the teeth inside the mouth; and a film indicator rotatably mounted with respect to the bite portion, rotated with the film support portion, and located outside the mouth. Since the bite portion is detached from the film support portion, an angle of the film support portion can be varied to correspond to various oral structures of patients. Since angle change of the film support portion can be recognized externally, an irradiation angle of a radiation center line can easily be adjusted.

## Description

### TECHNICAL FIELD

The present invention relates to a film holder located at a lingual side of the teeth inside the mouth to hold a film for periapical radiography, which obtains the radiographic image of the teeth and teeth supporting structures, and more particularly, to a film holder for periapical radiography, which can externally identify an angle of a film with respect to a longitudinal axis of the teeth inside the mouth with ease and also provides guidance for radiation beam angle.

### BACKGROUND ART

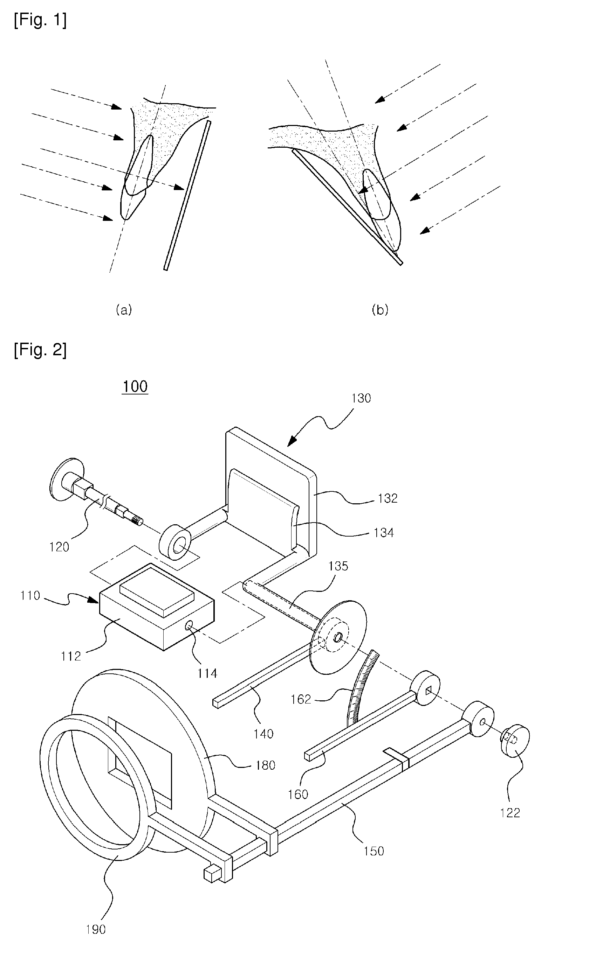

Examples of periapical radiography technique for dental procedure include the paralleling technique and the bisecting angle technique. FIG. 1 describes sectional view of both techniques according to the related art, in which (a) illustrates the paralleling technique and (b) illustrates the bisecting angle technique.

Referring to FIG. 1, the paralleling technique is to carry out radiography by locating a film inside the mouth in parallel with a longitudinal axis of the teeth. As the film is located in parallel with the longitudinal axis of the teeth, a teeth image obtained by parallel radiography has a one-to-one size corresponding to real teeth. However, since a space inside the mouth is small and low, it is difficult to locate the film in parallel with the longitudinal axis of the teeth. Also, in order to minimize distortion of the image, a radiation source may be spaced apart from the teeth far away if possible, and in order to maintain a certain distance between the radiation source and the teeth and guide a direction of central beam of radiation, a long cone of 12~16inches may be used.

The bisecting angle technique is to carry out radiography by aiming the central beam of x-ray in perpendicular to a bisector of an angle between the longitudinal axis of the teeth and the film. The bisecting angle technique is based on the principle that oblique sides at both sides of an isosceles triangle have the same length to each other. A teeth image obtained by the bisecting technique also has a one-to-one size corresponding to real teeth. Although the bisecting angle technique has relatively less limitation inside the mouth than the paralleling technique, a problem occurs in that a holding angle of the film may be varied randomly by an anatomical structure. Also, a problem occurs in that it is difficult to take the radiograph of the teeth accurately at recommended angles defined in a textbook.

In this respect, the US Patent No. 5,737,388 titled "DIGITAL INTRAORAL X-RAY PHOTOGRAPHY METHOD AND HOLDER FOR PICTURE PLATE OR X-RAY FILM" discloses three linked parts of a film holder, a bite portion and an alignment rod. However, there is provided a scale that can measure an angle, since the angle should be controlled before the bite portion is bitten by a patient, it is not substantially convenient.

The Korean Laid-Open Patent No. 2005-29047 titled "ALIGNMENT DEVICE FOR X-RAY PHTOGRAPHY BASED ON ANATOMICAL ORAL STRUCTURE" is intended to minimize pain by considering the oral structure of the human body. Although the alignment device for X-ray photography has a link structure that allows a ring and an alignment rod to be linked with each other in various manners in accordance with a tilt angle, problems occur in that several combinations defined structurally are only available and they cannot react with various oral structures of patients appropriately.

### DETAILED DESCRIPTION OF THE INVENTION

### TECHNICAL PROBLEMS

Accordingly, the present invention has been devised to obviate distortion of teeth supporting structures and the teeth on a radiograph, which occurs when an existing film holder having a bite portion and a film support portion of a fixed angle is used for patients having various oral structures.

An object of the present invention is to provide a film holder that can vary an angle of a film support portion to correspond to various oral structures of patients while a patient bites a bite portion of the film holder.

Another object of the present invention is to provide a film holder that can externally identify angle change of a film support portion and easily control the x-ray beam angle based on angle change of the film support portion, thereby allowing radiography more faithfully based on the bisecting angle technique.

Other object of the present invention is to provide a film holder that can be used for either the bisecting angle technique or the paralleling technique, can easily be used by a dentist and a dental hygienist, and can increase accuracy of a radiographic image of the teeth and teeth supporting structures.

### TECHNICAL SOLUTIONS

To achieve these objects and other advantages and in accordance with the purpose of the invention, as embodied and broadly described herein, according to one exemplary aspect of the present invention, a film holder located at a lingual side of the teeth inside the mouth for periapical radiography for obtaining the radiographic image of the teeth and teeth supporting structures inside the mouth to support a film comprises a bite portion temporarily fixed by the teeth; a film support portion rotatably mounted with respect to the bite portion and located at the lingual side of the teeth inside the mouth; and a film indicator rotatably mounted with respect to the bite portion, rotated with the film support portion, and located outside the mouth.

Although the bite portion is generally bitten by the teeth, if the teeth are lost, the bite portion may be fixed temporarily by another fixing means such as a modeling compound. If the bite portion is bitten by the teeth, it is perpendicular to a longitudinal axis of the teeth. The film support portion and the film indicator may be rotated together separately from the bite portion fixed by the teeth. The film support portion can locate a general film or digital film device for intraoral radiography, and the film indicator can recognize a rotational angle of the film support portion outside the mouth to correspond to pivot action of the film indicator. Although the film indicator may simply be provided in a rod shape vertically extended from an axle, it may be provided as a display portion having a rotational portion corresponding to rotation of the axle to display rotation of the rotational portion in a digitalized mode.

The bite portion is rotatably detached from the film support portion, and the rotational angle between the film support portion and the bite portion can be recognized outside the mouth. Accordingly, a user can directly read the rotational angle of the film support portion with respect to the bite portion inside the mouth, and can directly identify a vertex of an isosceles triangle based on the bisecting angle technique. For example, the user can read the angle between the bite portion and the film support portion through the film indicator and bite body indicator. At this time, an x-ray beam angle can directly be determined to be perpendicular to a bisector of an angle between the longitudinal axis of the teeth and the film.

In this case, the film holder further comprises a radiation angle indicator rotatably mounted on the same axle as that of the film support portion and the film indicator to display the x-ray beam angle.

In another aspect of the present invention, a film holder located at a lingual side of the teeth inside the mouth for periapical radiography for obtaining a radiographic image of the teeth and teeth supporting structures inside the mouth to support a film comprises a bite portion including a bite body temporarily fixed to the teeth, an axle hole passing through the bite body in a horizontal direction, and a central axle mounted in the axle hole; a film support portion rotatably mounted with respect to the central axle and located inside the mouth; a film indicator partially receiving the central axle, spaced apart from the bite body at a predetermined interval, and rotating with the film support portion by being physically linked with the film support portion; and a radiation angle indicator partially receiving the central axle near the film indicator, rotatably mounted based on the central axle independently from the film indicator.

In other aspect of the present invention, a film support portion mounted in a film holder, which is located at a lingual side of the teeth inside the mouth for periapical radiography for obtaining a radiographic image of the teeth and teeth supporting structures inside the mouth to support a film, comprises a film support plate corresponding to a digital film device and an elastic piece formed at one side of the film support plate, the film support plate being provided with a recess portion at the front to correspond to a link cable of the digital film device.

### ADVANTAGEOUS EFFECTS

According to the film holder of the present invention, an angle of a unique film support portion of a patient can be recognized outside the mouth to correspond to various oral structures of patients while the patient bites a bite portion of the film holder.

Also, angle change of the film support portion inside the mouth can directly be identified externally through an analog or digital film indicator. Since an angle of the film support portion can directly be identified externally, x-ray beam angle can quickly be specified and the radiation source can be arranged.

The present invention can be used for either the bisecting angle technique and the paralleling technique, can easily be used by a dentist and a dental hygienist, and can increase accuracy of a radiographic image.

### BRIEF DESCRIPTION OF THE DRAWINGS

The accompanying drawings, which are included to provide a further understanding of the invention and are incorporated in and constitute a part of this application, illustrate embodiment(s) of the invention and together with the description serve to explain the principle of the invention. In the drawings:
FIG. 1 is a sectional view for comparison of the paralleling technique and the bisecting angle technique according to the related art;
FIG. 2 is an exploded perspective view of a film holder according to one embodiment of the present invention;
FIG. 3 is a side sectional view illustrating a process of carrying out the bisecting angle technique by using a film holder of FIG. 2;
FIG. 4 is a partially enlarged view illustrating another film support portion similar to a film support portion of FIG. 2;
FIG. 5 is a partially enlarged view illustrating a film support portion according to another embodiment of the present invention;
FIG. 6 is a partially enlarged view illustrating a film support portion according to still another embodiment of the present invention;
FIG. 7 is a partially enlarged view illustrating a film support portion according to further still another embodiment of the present invention;
FIG. 8 is a front view illustrating a radiation block portion of FIG. 6; and
FIG. 9 is a front view illustrating an aiming guide of FIG. 2.

### BEST MODE FOR CARRYING OUT THE INVENTION

Reference will now be made in detail to the preferred embodiments of the present invention, examples of which are illustrated in the accompanying drawings. It is to be understood that the present invention is not limited by the embodiment only. Wherever possible, the same reference numbers will be used throughout the drawings to refer to the same or like parts. Under this rule, reference may be made referring to examples illustrated in other drawings, and description apparent to those skilled in the art or repeated parts will be omitted.

FIG. 2 is an exploded perspective view of a film holder according to one embodiment of the present invention.

Referring to FIG. 2, a film holder 100 is intended to locate a film at a lingual side of the teeth inside the mouth for periapical radiography for obtaining a radiographic image of the teeth and teeth supporting structures inside the mouth. The film holder 100 includes a bite portion 110, a film support portion 130, a film indicator 140, and a radiation angle indicator 150. The bite portion 110 is fixed between the teeth by a jaw closing action of a mandible, and the film support portion 130 and the film indicator 140 are moved together but independently moved with respect to the bite portion 110. A user can externally recognize an angle of the film support portion 130 with respect to the bite portion 110 by identifying angle relation between a bite body indicator 160 and the film indicator 140. To this end, the film support portion 130 and the film indicator 140 are rotated based on the same axle, a central axle 120 in this embodiment.

In more detail, the bite portion 110 includes a bite body 112 and the central axle 120. The bite body 112 is temporarily fixed between the teeth by the jaw closing action of the mandible, and can directly be detached from the teeth by a jaw opening action of the mandible. The bite body 112 is provided with an axle hole 114 passing through the bite body in horizontal direction, and the central axle 120 is provided to pass through the axle hole 114. The central axle 120 may partially have a circle shaped section as described later, or may have a non-circle shaped section or quadrangle section for fixation with another part.

The film support portion 130 is rotatably mounted in the bite body 112. In more detail, the film support portion 130 includes a film support plate 132 and an elastic piece 134, and both ends of the film support plate 132 are extended towards the bite body 112 to receive both sides of the bite body 112. In a state that the film support 130 receives both sides of the bite body 112, the central axle 120 passes through the extension portion of the film support plate 132 and the bite body 112, and the film support portion 130 can rotatably be mounted in the bite portion 110 based on the central axle 120.

One side of the film support portion 130 is extended from the bite body 112 in an axial direction along the central axle 120. At this time, the extension portion 135 of the film support portion 130 can be rotated around the central axle 120 while receiving the central axle 120. The film indicator 140 is extended in a rod shape from the end of the extension portion 135 of the film support portion 130. The film indicator 140 is spaced apart from the bite body 112 at a predetermined interval. For example, supposing that the bite body 112 is bitten by the teeth, the film indicator 140 is preferably spaced apart from the bite body 112 so as not to be disturbed by a face of a patient.

The end of the central axle 120 is exposed from the extended end of the film support portion 130, and a radiation angle indicator 150 and a bite body indicator 160 are assembled into the exposed portion. The central axle 120, engaged with the bite body indicator 160 partially, has a quadrangle section, and the end of the bite body indicator 160 has a quadrangle hole to correspond to the quadrangle section, whereby the bite body indicator can be fixed to the central axle 120. On the other hand, a circle hole is formed at the end of the radiation angle indicator 150 to correspond to the central axle 120, wherein the radiation angle indicator 150 can be rotated and fixed with respect to the central axle separately from the other indicators.

A thread may be formed at the end of the central axle 120. The user can control the coupling force among the film indicator 140, the radiation angle indicator 150 and the bite body indicator 160 by turning a cap 122 of the end of the central axle 120, and can mutually control a rotational angle among the indicators by releasing the cap 122. The user can fix the rotational angle among the indicators by tightening the cap 122. At this time, the film indicator 140, the radiation angle indicator 150 and the bite body indicator 160 are fixed by friction based on the central axle 120. According to another embodiment, in order to increase friction, another surface treatment may be carried out for the indicators, or radial protrusions may be formed around the central axle.

The film indicator 140 or the film support portion 130 may be provided with an angle scale for indicating an angle. The angle between the bite body indicator 160 and the film indicator 140 can be measured through the angle scale. Also, the angle between the bite portion 110 and the film support portion 130 can indirectly be measured in situ through the angle scale. The bite portion 110 or the bite body indicator 160 may also be provided with a scale 162 for measuring an angle.

Although the film indicator 140 is simply provided in a rod shape extended from the central axle 120 in a vertical direction in this embodiment, it may be provided as a display portion having a rotational portion corresponding to rotation of the central axle to display rotation of the rotational portion in a digitalized mode by sensing and digitizing the rotation of the rotational portion.

The radiation angle indicator 150 is intended to predict and guide x-ray beam angle. In this embodiment, the radiation angle indicator 150 is provided in a rod shape. In this case, a radiation block portion 180 and an aiming guide 190 are slidably mounted on the radiation angle indicator 150. The radiation block portion 180 allows the radiation to pass through a hole only, whereby a face or body of a patient can be prevented from being unnecessarily exposed to the radiation. Also, the aiming guide 190 is intended for the positioning of the irradiation portion of the radiation source. A dentist or a dental hygienist can simply locate the radiation source by moving and adjusting the irradiation portion of the equipment to the aiming guide 190. Various means for adjusting the angle and direction of x-ray beam may be used.

Since the radiation block portion 180 and the aiming guide 190 can be slid along the radiation angle indicator 150, their location can easily adjusted. Also, it is very advantageous in that the radiation block portion 180 and the aiming guide 190 can detachably be provided.

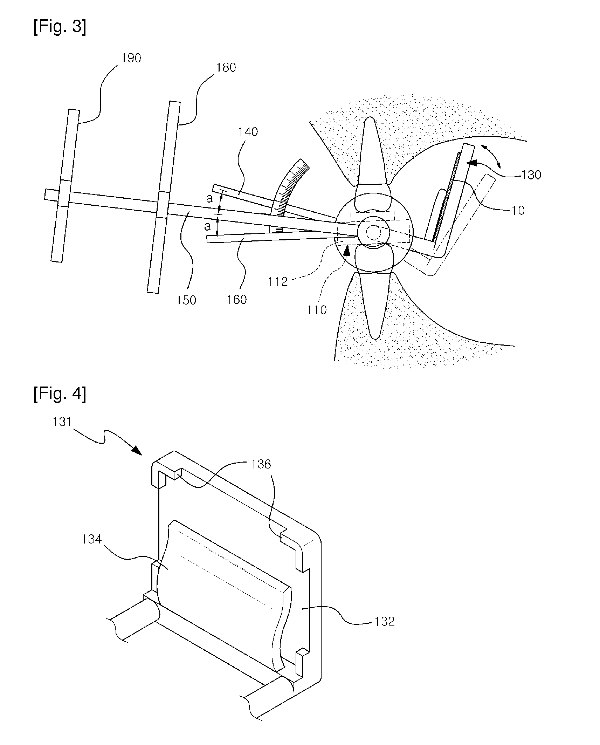

FIG. 3 is a side sectional view illustrating a process of carrying out the bisecting angle technique by using a film holder of FIG. 2.

Referring to FIG. 3, after mounting a film 10 in the film support portion 130 of the film holder 100, a user such as a dentist or a dental hygienist locates the film support portion 130 inside the mouth of a patient and allows the patient to bite the bite body 112 of the bite portion 110 with the teeth. Since the bite body 112 is bitten by the teeth up and down, it can be maintained in perpendicular to the longitudinal axis of the teeth. It is supposed that the bite body 112 is fixed to the teeth in perpendicular to the longitudinal axis of the teeth.

The user can find an optimized angle by rotating the film indicator 140, exposed to the outside, up and down. Preferably, the film indicator 140 is rotated such that the longitudinal axis of the teeth is almost parallel with the film, whereby distortion caused by the bisecting angle technique can be more minimized. At this time, the angle of the film support portion 130 may be varied depending on the oral structure of the patient, the selected film support plate, etc. Since the film support portion 130 is rotated up and down together with the film indicator 140, it can be located appropriately inside the mouth of the patient.

If the most appropriate location of the film indicator 130 is determined, the angle between the bite body indicator 160 and the film indicator 140 can be read through the scale. The user can easily determine the x-ray beam angle by referring to the angle between the bite body indicator 160 and the film indicator 140. As described above, according to the bisecting angle technique, after measuring the angle 2a between the bite body indicator 160 and the film indicator 140, the user can adjust the angle 2a such that the bite body indicator 160 or the film indicator 140 is spaced apart from the radiation angle indicator 150 as much as the bisected angle 'a'.

According to the bisecting angle technique, the angle parallel with the radiation angle indicator 150 may be regarded as the angle based on the bisecting angle technique. The user can adjust the x-ray beam angle by referring to the radiation angle indicator 150.

FIG. 4 is a partially enlarged view illustrating another film support portion similar to a film support portion of FIG. 2.

Referring to FIG. 4, the film support portion 131 includes a film support plate 132 and an elastic piece 134 formed at one side of the film support plate 132. Generally, a radiation sensitive film is used for periapical radiography for obtaining a radiographic image of the teeth and teeth supporting structures inside the mouth. The size of the film support plate 132 is provided considering the size of the film. The elastic piece 134 is formed from one side of the film support plate 132 in a single body with the film support plate 132, and can temporarily fix the film inside the mouth.

Also, in order to restrict flickering of the film, an edge guide 136 may be formed at a side or edge of the film support plate 132. The edge guide 136 is formed to correspond to the shape of the film, which is inserted thereto, and its one side may have an interval wider than that of the other side by considering insertion of the film.

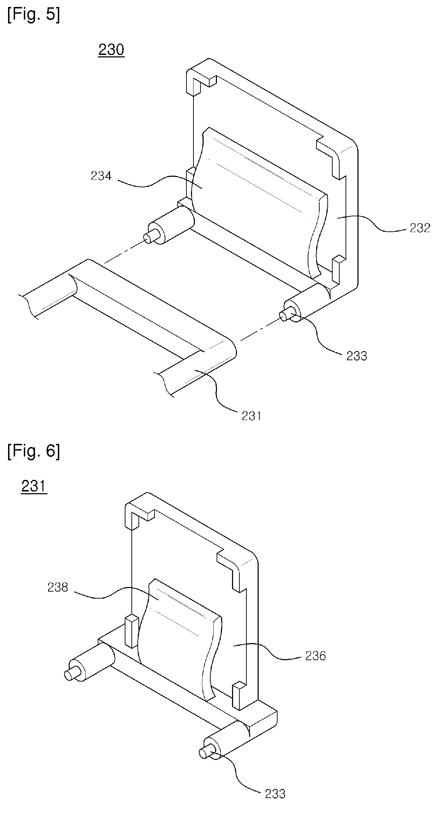

FIG. 5 is a partially enlarged view illustrating a film support portion according to another embodiment of the present invention.

Referring to FIG. 5, the film support portion 230 includes a film support plate 232 and an elastic piece 234 formed at one side of the film support plate 232. The film support portion 230 is detachably mounted in a portion 231 screwed with the bite portion. Two protrusions 233 are formed below the film support plate 232, and grooves are formed in the portion 231 to be screwed with the bite portion, to correspond to the protrusions 233.

The film used is radiation sensible" and the size of the film support plate 232 may also be provided depending on the size of the film. Even though a digital film device is used, the film support plate may be detachably mounted in the portion screwed with the bite portion.

When maxillary teeth, mandibular teeth, anterior teeth or molar teeth are subjected to radiography, an inserting direction or the size of the film may be varied. In this case, the film support plate can be exchanged with another one, whereby an appropriate film support plate can be used depending on the location of the teeth subjected to radiography and an oral structure around the teeth.

FIG. 6 is a partially enlarged view illustrating a film support portion according to still another embodiment of the present invention.

Referring to FIG. 6, the film support portion 231 may be formed in various shapes. For example, the film support portion 231 includes a longitudinal film support plate 236 and an elastic piece 238 formed at one side of the film support plate 236. The longitudinal film support plate is located at a lingual side of front teeth and can be used for radiography. Two protrusions 233 are formed below the film support plate 236 to exchange the film support plate 236 with another one. The film support plate 236 is screwed with the other portion of the bite portion, to correspond to the protrusions 233. In the same manner as FIG. 5, even though a digital film device is used, the film support plate may be detachably mounted in the portion screwed with the bite portion.

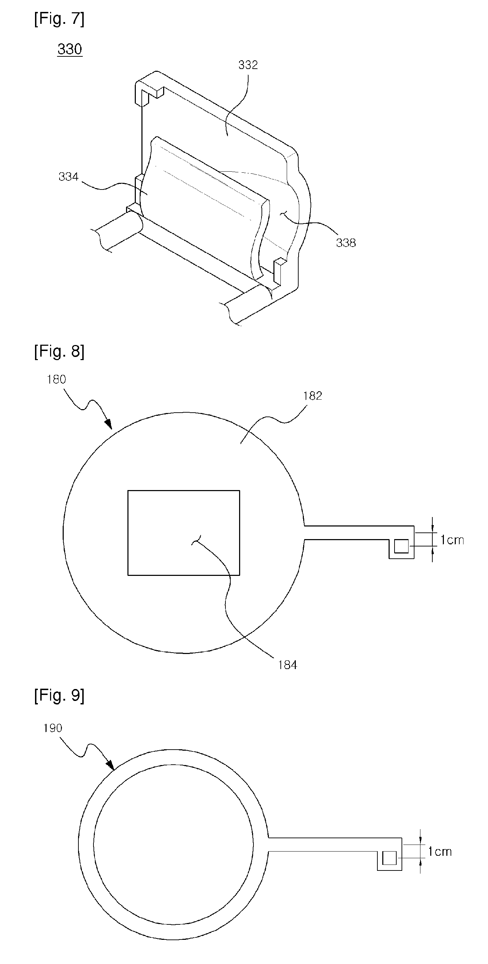

FIG. 7 is a partially enlarged view illustrating a film support portion according to further still another embodiment of the present invention.

Referring to FIG. 7, the film support portion 330 includes a film support plate 332 and an elastic piece 334 formed at one side of the film support plate 332. A recess portion 338 is formed at one side in front of the film support plate 332. Generally, a digital film device may be provided with a protruded portion to be connected with an external cable. The recess portion 338 is provided in front of the film support plate 332 to receive the protruded portion.

As described above, even though the digital film device is used, the film support plate may be detachably mounted in other portion. The size of the film support plate can be provided considering the size of the film. Also, in order to fix the digital film device, an edge guide may be formed at a side or edge of the film support plate.

FIG. 8 is a front view illustrating a radiation block portion of FIG. 6, and FIG. 9 is a front view illustrating an aiming guide of FIG. 2.

Referring to FIG. 8, the radiation block portion 180 is slidably mounted in the radiation angle indicator 150, and includes a blocking plate 182 provided with a radiation pass hole 184. The blocking plate 182 is spaced apart from the radiation angle indicator 150 at a distance to reach the bite portion 110, and can reduce radiation exposure of the other body except the teeth and teeth supporting structures of interest.

Also, in order to compensate the distance from the center of the axle hole of the bite portion to the upper end of the bite portion or the center of the teeth and teeth supporting structures subjected to radiography, the center of the blocking plate 182 can be located to be higher than the hole for the radiation angle indicator 150 at a certain level. In this embodiment, the center of the blocking plate 182 is located to be higher than the radiation angle indicator 150 as much as 1cm, approximately. As a result, the center of the teeth subjected to radiography can correspond to the center of the pass hole 184.

Referring to FIG. 9, the aiming guide 190 is also located to be higher than the hole for the radiation angle indicator 150 at a certain level. Likewise, the radiation center line can correspond to the center of the teeth and teeth supporting structures subjected to radiography.

It will be apparent to those skilled in the art that the present invention can be embodied in other specific forms without departing from the spirit and essential characteristics of the invention. Thus, the above embodiments are to be considered in all respects as illustrative and not restrictive. The scope of the invention should be determined by reasonable interpretation of the appended claims and all change which comes within the equivalent scope of the invention are included in the scope of the invention.

### INDUSTRIAL APPLICABILITY

The film holder of the present invention is intended for various oral structures of patients. Accordingly, while the patient bites the bite portion of the film holder, the angle of the unique film support portion can be recognized externally.

The present invention can be used for either the bisecting angle technique or the paralleling technique, can easily be used by a dentist and a dental hygienist, and can increase accuracy of a radiographic image.

## Claims

1. A film holder located at a lingual side of the teeth inside the mouth to support a film for periapical radiography, which obtains the radiographic image of the teeth and teeth supporting structures, the film holder comprising:
a bite portion temporarily fixed to the teeth;
a film support portion rotatably mounted with respect to the bite portion and located at the lingual side of the teeth inside the mouth; and
a film indicator rotatably mounted with respect to the bite portion, rotated with the film support portion by being linked with the film support portion,
and located outside the mouth.

2. The film holder of claim 1, wherein the bite portion or the film indicator is provided with an angle scale for identifying an angle of the film indicator.

3. The film holder of claim 1, further comprising a bite body indicator for displaying a fixed angle of the bite portion to correspond to the film indicator.

4. The film holder of claim 1, further comprising a radiation angle indicator rotatably mounted on the same axle as that of the film support portion and the film indicator and rotated independently from the film support portion and the film indicator.

5. The film holder of claim 4, wherein the radiation angle indicator is provided with an aiming guide for adjustment of x-ray beam angle, the aiming guide being slidably mounted in the radiation angle indicator.

6. The film holder of claim 4, wherein the radiation angle indicator is provided with a radiation block portion for allowing radiation to pass through a specific portion only, the radiation block portion being slidably mounted in the radiation angle indicator.

7. The film holder of claim 6, wherein the radiation block portion includes a blocking plate provided with a radiation pass hole and spaced apart from the radiation angle indicator at a distance to reach the bite portion, and the center of the blocking plate is higher than the radiation angle indicator.

8. The film holder of claim 1, wherein the film support portion includes a film support plate and an elastic piece formed at one side of the film support plate, the film support plate being provided with an edge guide protruded to correspond to an edge of a film mounted in the film support plate.

9. The film holder of claim 1, wherein the film support portion includes a film support plate corresponding to a digital film device and an elastic piece formed at one side of the film support plate, the film support plate being provided with a recess portion at a front to correspond to a link cable of the digital film device.

10. A film holder located at a lingual side of teeth inside the mouth to support a film for periapical radiography, which obtains a radiographic image of the teeth and teeth supporting structures, the film holder comprising:
a bite portion including a bite body fixed by the teeth, an axle hole passing through the bite body in a horizontal direction, and a central axle mounted in the axle hole;
a film support portion rotatably mounted with respect to the central axle and located inside the mouth;
a film indicator partially receiving the central axle, spaced apart from the bite body at a predetermined interval, rotated with the film support portion by being physically linked with the film support portion; and a radiation angle indicator partially receiving the central axle near the film indicator, rotatably mounted based on the central axle independently from the film indicator.

11. The film holder of claim 10, wherein the bite portion or the film indicator is provided with an angle scale for identifying an angle of the film indicator.

12. The film holder of claim 10, further comprising a bite body indicator for displaying a fixed angle of the bite portion to correspond to the film indicator, the bite body indicator being fixed to the bite body.

13. The film holder of claim 10, wherein the radiation angle indicator is provided with a radiation block portion for allowing radiation to pass through a specific portion only and an aiming guide for adjustment of a radiation center line, the radiation block portion and the aiming guide being slidably mounted in the radiation angle indicator.

14. The film holder of claim 10, wherein the film support portion includes a film support plate and an elastic piece formed at one side of the film support plate, the film support plate being provided with an edge guide protruded to correspond to an edge of a film mounted in the film support plate.

15. The film holder of claim 10, wherein the film support portion includes a film support plate corresponding to a digital film device and an elastic piece formed at one side of the film support plate, the film support plate being provided with a recess portion at a front to correspond to a link cable of the digital film device.

16. A film support portion installed in a film holder located at a lingual side of teeth inside the mouth to support a film for periapical radiography, which obtains a radiographic image of the teeth and teeth supporting structures, the film support portion comprising:
a film support plate corresponding to a digital film device and an elastic piece formed at one side of the film support plate, the film support plate being provided with a recess portion at a front to correspond to a link cable of the digital film device.

17. The film support portion of claim 16, wherein the film support plate includes an edge guide protruded to correspond to an edge of a film mounted in the film support plate.
